Europäisches Patentamt

European Patent Office

Office européen des brevets

(19)

(11) Veröffentlichungsnummer: **0 118 778**
**B1**

(12) # EUROPÄISCHE PATENTSCHRIFT

(45) Veröffentlichungstag der Patentschrift:
03.06.87

(51) Int. Cl.⁴: **A 61 B 17/58**

(21) Anmeldenummer: **84101380.8**

(22) Anmeldetag: **10.02.84**

(54) **Verriegelungsnagel.**

(30) Priorität: **09.03.83 DE 8306675 U**

(43) Veröffentlichungstag der Anmeldung:
**19.09.84 Patentblatt 84/38**

(45) Bekanntmachung des Hinweises auf die Patenterteilung:
**03.06.87 Patentblatt 87/23**

(84) Benannte Vertragsstaaten:
**CH DE FR GB LI**

(56) Entgegenhaltungen:
**EP - A - 0 008 758**
**DE - A - 3 003 957**
**FR - A - 2 342 710**
**FR - A - 2 387 637**
**FR - A - 2 452 914**
**GB - A - 2 114 005**

(73) Patentinhaber: **Howmedica International, Inc.**
**Zweigniederlassung Kiel,**
**Professor-Küntscher-Strasse 1-5, D-2301 Schönkirchen**
**üb. Kiel (DE)**

(72) Erfinder: **Richter, Karl Manfred, Haferkamp 14,**
**D-2304 Wendtorf (DE)**

(74) Vertreter: **Dipl.-Ing. H. Hauck Dipl.-Phys. W. Schmitz**
**Dipl.-Ing. E. Graalfs Dipl.-Ing. W. Wehnert Dr.-Ing. W.**
**Döring, Neuer Wall 41, D-2000 Hamburg 36 (DE)**

ACTORUM AG

**Beschreibung**

Die Erfindung bezieht sich auf einen Verriegelungsnagel zum Eintreiben in einen Knochenkanal zur Versorgung von Knochenfrakturen mit einem länglichen hohlen Körper, der am vorderen Ende verjüngt ist und am Einschlagende eine Erweiterung aufweist zur Aufnahme eines Einschlagwerkzeugs und mindestens ein Paar Querbohrungen aufweist zur Aufnahme einer Knochenschraube. Ein derartiger Verriegelungsnagel ist aus der Fr-A-2 387 637 bekannt geworden. Mit Hilfe eines geeigneten Zielgeräts, beispielsweise über einen Bildwandler, wird die Lage von Querbohrungen des Verriegelungsnagels im Knochenkanal ermittelt, so dass im Knochen in Übereinstimmung mit diesen Querbohrungen Löcher im Knochen geformt werden, über die dann Knochenschrauben in den Knochen und den Verriegelungsnagel eingeschraubt werden. Auf diese Weise kann der Verriegelungsnagel auf beiden Seiten der Fraktur mit dem Knochen fest verbunden werden. Verriegelungsnägel dienen insbesondere der Versorgung von Trümmerbrüchen, die einen Knochen äusserst rotationsinstabil machen.

Bekannte Verriegelungsnägel haben ein sogenanntes Kleeblattprofil und einen sich über die grösste Länge des Nagelkörpers erstreckenden Längsschlitz, wobei nur die Enden des Nagels ein geschlossenes Profil darstellen. Es hat sich jedoch gezeigt, dass ein geschlitzter Nagel eine gewisse Torsion zulässt und vor allem relativ scharfe Kanten aufweist, die zu einer Verletzung und damit zu einer Perforationsgefahr für den Knochen führen können.

Andere herkömmliche Knochenmarknägel werden in den Knochenkanal eingeschlagen, ohne durch zusätzliche Hilfsmittel im Knochen fixiert zu werden. (z.B. sogenannter Küntscher-Nagel). Eine gewisse Rotationsstabilität wird mit Hilfe eines derartigen Knochennagels dadurch erreicht, dass er fest im Markraum eingespannt ist. Die Rotationsstabilität eines derartigen Knochennagels wird bei einem nach der EP-A-0 008 758 dadurch verbessert, dass an seinem Umfang in Achsrichtung Längsnuten geformt sind, die Schneiden bilden, die sich in die Knochenwand eingraben und dadurch eine Relativdrehung zwischen Nagel und Knochen verhindern.

Der Erfindung liegt die Aufgabe zugrunde, einen Verriegelungsnagel zu schaffen, der sich durch eine hohe Rotationsstabilität auszeichnet, den Knochen jedoch nicht beeinträchtigt.

Diese Aufgabe wird erfindungsgemäss dadurch gelöst, dass das Querschnittsprofil über seinen Umfang ringförmig geschlossen ist und der Radius der Aussenfläche über den Umfang sich verändert, derart, dass sich eine stetig abgerundete Kontur ergibt.

Bei einem Verriegelungsnagel erfolgt, wie oben bereits beschrieben, der Kraftschluss zwischen Knochen und Verriegelungsnagel bezüglich der Torsion über die quer verlaufenden Knochenschrauben. Die beim erfindungsgemässen Verriegelungsnagel von der Kreisbahn abweichende Aussenkontur des Nagels ermöglicht einen mehr oder weniger ausgeprägten Eingriff im Knochenkanal, um eine Relativdrehung zwischen Knochen und Nagel zu vermeiden, ohne jedoch eine Verletzung oder gar eine Perforation des Knochens herbeizuführen. Das ringförmig geschlossene Querschnittsprofil des neuerungsgemässen Nagels weist ein hohes Widerstandsmoment auf und ergibt mithin eine hohe Rotationsstabilität.

Bei einer Ausgestaltung der Erfindung ist vorgesehen, dass das Querschnittsprofil über den Umfang abwechselnd Erhebungen und Vertiefungen aufweist. Wie bereits erwähnt, müssen die Übergänge zwischen Erhebung und Vertiefung stetig und möglichst weich sein, um die Verletzungsgefahr zu vermindern.

In einer Ausgestaltung der Erfindung ist hierzu vorgesehen, dass die radial am weitesten aussen und am weitesten innen liegenden Punkte der Erhebungen und Vertiefungen auf einem Kreisbogen konzentrisch zur Achse liegen. Eine derartige Ausführung kann z.B. nach einer weiteren Ausgestaltung der Erfindung drei im Umfang gleichmässig beabstandete kreisförmige Erhebungen aufweisen, zwischen denen relativ flache gerundete Vertiefungen liegen.

Verriegelungsnägel der erfindungsgemässen Art weisen einen Durchmesser von 9 bis 18 mm auf und werden zumeist mit Hilfe eines sogenannten Führungsspiesses eingetrieben, der zuvor in den Knochenkanal eingebracht wurde. Zu diesem Zweck muss der Verriegelungsnagel einen in Längsrichtung durchgehenden Kanal für den Führungsspiess aufweisen. Das Material des erfindungsgemässen Verriegelungsnagels besteht naturgemäss aus körperverträglichem, nicht korrodierendem Material. Es ist gleichzeitig geeignet, im Strangpressverfahren hergestellt zu werden. Das Strangpressverfahren verhindert die Entstehung von Nähten oder Graten am Umfang des Nagels, welche äusserst unerwünscht sind.

Ausführungsbeispiele der Erfindung werden nachfolgend anhand von Zeichnungen näher erläutert.

Fig. 1 zeigt einen Schnitt durch einen Verriegelungsnagel, der in einem Oberschenkelknochen implantiert ist.

Fig. 2 zeigt einen Schnitt durch einen Verriegelungsnagel nach der Neuerung.

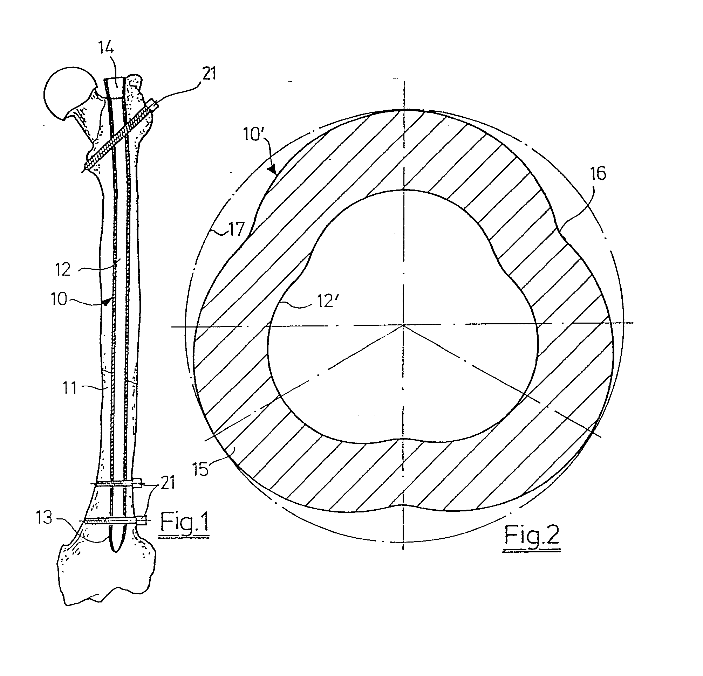

In Fig. 1 ist ein Verriegelungsnagel 10 vom Trochanter major in einen Oberschenkelknochen 11 eingetrieben. Der Verriegelungsnagel 10 ist ein Hohlprofil mit einem durchgehenden inneren Kanal 12, der bis zur verjüngten Spitze 13 des Nagels 10 reicht. Auf diese Weise kann bei Verwendung eines Führungsspiesses der Nagel 10 dem Führungsspiess entlang eingetrieben werden (wie dies in Verbindung mit der Nagelung nach Küntscher allgemein bekannt ist). Am Einschlagende weist der Nagel 10 eine konische Erweiterung 14 auf mit einem Innengewinde, mit dem ein Einschlagwerkzeug in Eingriff gebracht werden kann.

Die Nagelungsmethode erfordert, dass nach dem Eintreiben des Verriegelungsnagels 10 die Lage von Paaren von Querbohrungen mit Hilfe eines Bildwandlers ermittelt wird, um in der gemeinsamen Achse eines Bohrungspaares ein Loch in die Cortikalis zu bohren zwecks Einschraubens von Querschrauben 21. Bei der Darstellung nach Fig. 1 sind beidseits der Trümmerfraktur jeweils Querknochenschrauben 21 eingeschraubt. Die oberste ist dabei schräg eingesetzt, entsprechend den hierfür vorgesehenen Bohrungen im Nagel 10.

Der Nagel 10 kann im Querschnitt ein Profil haben, wie es in Fig. 2 dargestellt ist. Der Nagel 10' hat im Querschnitt ein «Kleeblattprofil» mit drei im Umfangsabstand von 120° beabstandeten Erhebungen 15, dessen Aussenkontur kreisbogenförmig ist, wobei sich die Kreisbögen über einen Winkel von etwa 90° erstrecken. Zwischen den Erhebungen 15 befinden sich Vertiefungen 16 oder Nuten, wobei die Erhebungen 15 in die Vertiefungen 16 in einem gerundeten weichen Übergang übergehen, wie in Fig. 2 dargestellt.

Die Wand des Nagels 10 ist über den Umfang gleichmässig dick, so dass der Kanal 12' eine Innenkontur hat, die der Aussenkontur des Nagels 10' entspricht. Die Erhebungen liegen mit ihren höchsten Punkten auf einem gemeinsamen Kreisbogen 17. Entsprechendes gilt für die Vertiefungen 16. Der gezeigte Nagel weist ein hohes Widerstandsmoment auf bei relativ geringem Materialaufwand.

### Patentansprüche

1. Verriegelungsnagel zum Eintreiben in einen Knochenkanal zur Versorgung von Knochenfrakturen mit einem länglichen hohlen Körper, der am vorderen Ende (13) verjüngt ist und am Einschlagende eine Erweiterung (14) aufweist zur Aufnahme eines Einschlagwerkzeugs und mindestens ein Paar Querbohrungen aufweist zur Aufnahme einer Knochenschraube (21), dadurch gekennzeichnet, dass das Querschnittsprofil über seinen Umfang ringförmig geschlossen ist und der Radius der Aussenfläche über den Umfang sich verändert, derart, dass sich eine stetig abgerundete Kontur ergibt.

2. Verriegelungsnagel nach Anspruch 1, dadurch gekennzeichnet, dass das Querschnittsprofil über den Umfang abwechselnd Erhebungen (15) und Vertiefungen (16) aufweist.

3. Verriegelungsnagel nach Anspruch 2, dadurch gekennzeichnet, dass die radial am weitesten aussen und am weitesten innen liegenden Punkte der Erhebungen (15) und Vertiefungen (16) auf einem Kreisbogen (17) konzentrisch zur Achse liegen.

4. Verriegelungsnagel nach Anspruch 3, gekennzeichnet durch drei im Umfang gleichmässig beabstandete kreisbogenförmige Erhebungen (15), zwischen denen relativ flach gerundete Vertiefungen (16) liegen.

5. Verriegelungsnagel nach einem der Ansprüche 1 bis 4, dadurch gekennzeichnet, dass seine Wand über den Umfang annähernd gleich dick ist.

### Claims

1. Interlocking nail adapted to be driven into a medullary canal for the treatment of bone fractures, comprising an elongated hollow body which is tapered on the front end (13) and at the end of driving-in is provided with an enlargement (14) for the accommodation of a tool for driving-in and has at least one pair of transverse bores for the accommodation of a bone screw (15), characterized in that the cross sectional profile over the circumference thereof is closed in the shape of a ring, and the radius of the outer surface over the circumference changes in such a manner that a steadily rounded contour results.

2. Interlocking nail according to claim 1, characterized in that the cross sectional profile over the circumference comprises alternating elevations (18) and deepenings (16).

3. Interlocking nail according to claim 2, characterized in that the sites of the elevations (18) and deepenings (16) disposed radially farthest outward and farthest inward lie on a circular arc (17) concentric with respect to the axis.

4. Interlocking nail according to claim 3, characterized by three circular arc-shaped elevations (18) spaced uniformly on the circumference with relatively flat-rounded deepenings (16) disposed therebetween.

5. Interlocking nail according to any one of the claims 1 to 4, characterized in that the wall thereof over the circumference approximately is of a uniform thickness.

### Revendications

1. Tige d'ancrage destinée à être enfoncée dans un canal médullaire pour la réduction de fracture, ayant un corps creux allongé qui est aminci à son extrémité avant (13), comporte à son extrémité d'enfoncement un élargissement (14) destiné à recevoir un outil d'enfoncement et présente au moins une paire de trous transversaux destinée à recevoir une vis à os (21), caractérisée en ce que sa section forme un anneau fermé et que le rayon de sa surface extérieur varie le long de la circonférence de façon telle qu'il a un contour partout arrondi.

2. Tige d'ancrage selon la revendication 1, caractérisée en ce que le profil de sa section présente alternativement des éminences (15) et des creux (16) dans la direction circonférentielle.

3. Tige d'ancrage selon la revendication 2, caractérisée en ce que les points radialement le plus à l'extérieur et le plus à l'intérieur des éminences (15) et des creux (16) sont sur un cercle (17) centré sur l'axe.

4. Tige d'ancrage selon la revendication 3, caractérisée par trois éminences en arc de cercle (15) également espacées dans la direction circonférentielle et entre lesquelles se trouvent des creux arrondis (16) relativement peu profonds.

5. Tige d'ancrage selon l'une des revendications 1 à 4, caractérisée en ce que sa paroi a sur toute la circonférence une épaisseur à peu près constante.

Fig.1

Fig.2